Europäisches Patentamt

European Patent Office

Office européen des brevets

(11) Veröffentlichungsnummer: **0 469 482 A1**

# (12) EUROPÄISCHE PATENTANMELDUNG

(21) Anmeldenummer: **91112597.9**

(22) Anmeldetag: **26.07.91**

(51) Int. Cl.5: **C07C 53/126**, C07C 51/41, C08K 3/24, C11D 13/02

(30) Priorität: **01.08.90 DE 4024414**

(43) Veröffentlichungstag der Anmeldung:
**05.02.92 Patentblatt 92/06**

(84) Benannte Vertragsstaaten:
**BE DE ES FR GB IT LU NL**

(71) Anmelder: **HOECHST AKTIENGESELLSCHAFT
Postfach 80 03 20
W-6230 Frankfurt am Main 80(DE)**

(72) Erfinder: **Heinrichs, Franz-Leo, Dr.
Am Arenberg 20
W-8901 Gablingen(DE)**
Erfinder: **Piesold, Jan-Peter, Dr.
Oblatterwallstrasse 44
W-8900 Augsburg(DE)**

(54) **Verfahren zur Reinigung von Salzen langkettiger aliphatischer Carbonsäuren und deren Verwendung als Kunststoffaditive.**

(57) Verfahren zur Reinigung von Salzen langkettiger aliphatischer Carbonsäuren, insbesondere von Montansäuren, dadurch gekennzeichnet, daß man die rohen Salze mit geeigneten organischen Lösungsmitteln, insbesondere Methylethylketon, extrahiert.

EP 0 469 482 A1

Die vorliegende Erfindung betrifft ein Verfahren zur Reinigung von (technischen) Salzen langkettiger aliphatischer Carbonsäuren und die Verwendung der so gereinigten Salze als Kunststoffadditive.

Salze der Fettsäuren mit Metallen der I., II. und III. Hauptgruppe sowie der II. Nebengruppe der Periodensystems der Elemente besitzen große Bedeutung als Kunststoffadditive. Sie werden überwiegend als Gleit- und Trennmittel eingesetzt. Weiterhin dienen sie aufgrund ihres Neutralisationsvermögens gegenüber Mineralsäuren als Stabilisatoren in säureempfindlichen Kunststoffen sowie als Nukleierungsmittel in teilkristallinen Kunststoffen (T. Riedel "Gleitmittel", Zeitschrift Kunststoffe 77, (1987), 10, S.1081-1084).

Obwohl in den meisten der oben genannten Fälle die Metallsalze von natürlich vorkommenden Fettsäuren Verwendung finden, kann es bei Kunststoffen, die bei hohen Temperaturen verarbeitet werden, vorteilhaft sein, Metallsalze von Carbonsäuren mit größeren Kettenlängen einzusetzen. So kann z.B. bei der Herstellung von Großhohlkörpern aus hochmolekularem Polyethylen nach dem Extrusionsblasverfahren das Abdampfen von freigesetzten Fettsäuren zur Porenbildung im Material führen. Deshalb wird hier Calciumstearat zunehmend durch Calciummontanat ersetzt. Natrium- und Lithiummontanat zeigen in Polyestern und Polyamiden eine nukleierende Wirkung und erleichtern die Verarbeitung dieser Kunststoffe.

Bei den wie beschrieben vorteilhaft einsetzbaren Salzen von langkettigen aliphatischen Carbonsäuren handelt es sich bevorzugt um solche, welche die Metalle der I., II. oder III. Hauptgruppe bzw. der II., VII. oder VIII. Nebengruppe des Periodensystems der Elemente mit gesättigten, geradkettigen oder verzweigten $(C_{24}-C_{40})$-Mono- oder Dicarbonsäuren bilden. Auch Salzgemische - verschiedene Säuren und/oder verschiedene Metalle - erfüllen den genannten Zweck. Von besonderer Bedeutung sind die Salze der Elemente der I. oder II. Hauptgruppe bzw. der II. Nebengruppe des Periodensystems, insbesondere die Lithium-, Natrium-, Calcium- oder Zinksalze.

Von den in Frage kommenden Carbonsäuren, die meist aus natürlichen Rohstoffen gewonnen werden, sind aufgrund ihrer guten Verfügbarkeit die sogenannte Montansäure bzw. deren Salze mit den obengenannten Metallen von großer Bedeutung, besonders Lithium-, Natrium-, Calcium- oder Zinkmontanat oder deren Gemische, insbesondere Calciummontanat. Die Herstellung der Salze erfolgt nach bekannten Verfahren, z.B. durch Verseifung der entsprechenden Säuren mit Metalloxiden. Bei der Montansäure (oder auch Montansäuren genannt) handelt es sich um ein Gemisch aus überwiegend gesättigten aliphatischen Monocarbonsäuren mit Kettenlängen von 24-36 C-Atomen, das aus bitumenreicher Braunkohle über die Zwischenstufe des Rohmontanwachses gewonnen wird. Die Zusammensetzung des Gemisches ist abhängig vom verwendeten Rohstoff und dem Aufarbeitungsverfahren (vgl. Ullmanns Encyklopädie der technischen Chemie, 4. Auflage, Band 24, S. 16-21). Demzufolge ist z.B. unter dem Begriff Calciummontanat ein Salzgemisch (Säurereste mit verschiedenen Kettenlängen) zu verstehen.

Bedingt durch den Rohstoff und die Herstellung enthalten die technische Montansäure und damit auch ihre Folgeprodukte in Abhängigkeit vom Raffinationsgrad unterschiedliche Mengen an unerwünschten Begleitstoffen, deren Anteil für einige Typen bei etwa 20 % liegen kann.

Es handelt sich hierbei überwiegend um nicht zur Salzbildung befähigte organische Stoffe wie z.B. Reste der Nativester oder Kohlenwasserstoffwachse. Da diese Begleitstoffe meist keinen Beitrag zur beabsichtigten Wirkung der Metallmontanate als Kunststoffadditive leisten, sollten sie möglichst vollständig von den eigentlichen Salzen abgetrennt werden. Darüber hinaus haben die Salze der technischen Montansäuren, wie auch die Salze anderer langkettiger technischer Carbonsäuren, häufig den Nachteil, daß sie verfärbende Verunreinigungen enthalten. Diese bedingen ein schlechtes Farbverhalten der Montanate bei ihrer Anwendung als Kunststoffadditive, d.h. die so hergestellten Kunststoffprodukte sind verfärbt (Gelbfärbung).

Es wurde überraschenderweise gefunden, daß sich durch ein sehr einfach zu handhabendes Reinigungsverfahren die unerwünschten Begleitstoffe aus technischen Salzen langkettiger aliphatischer Carbonsäuren, insbesondere den Salzen der Montansäure, weitgehend entfernen lassen.

Gegenstand der vorliegenden Erfindung ist daher ein Verfahren zur Reinigung von Salzen langkettiger aliphatischer Carbonsäuren, dadurch gekennzeichnet, daß man die rohen Salze mit einem organischen Lösungsmittel extrahiert.

Für die Extraktion der rohen Salze kommen alle Lösungsmittel in Frage, die bevorzugt nichtionische, organische Verbindungen zu lösen vermögen. Hierzu zählen niedermolekulare $(C_1-C_3)$-Monoalkohole, Ester, Ketone, halogenierte aliphatische Kohlenwasserstoffe, aromatische und aliphatische Kohlenwasserstoffe oder Mischungen aus den beschriebenen Lösungsmitteln. Vorteilhaft ist die Verwendung solcher Lösungsmittel, die sich infolge ihres niedrigen Siedepunkts leicht restlos aus dem extrahierten

Salz entfernen lassen, wie z.B. Methanol, Ethanol, Ethylacetat, Aceton, Methylethylketon, Methylenchlorid, Chloroform oder halogenfreie Kohlenwasserstoffe im Siedebereich von 40 bis 100 $^\circ$C. Die bevorzugten Extraktionsmittel haben einen Siedepunkt im Bereich von 30 bis 100 $^\circ$C. Besonders bevorzugt ist die Verwendung von Methylethylketon.

Die Durchführung der Extraktion kann nach bekannten Verfahren sowohl kontinuierlich als auch diskontinuierlich erfolgen. Besonders bevorzugt sind kontinuierliche Extraktionsverfahren, z.B. auf Bandfiltern, bei denen das feingepulverte rohe Salz durch Berieseln mit Lösungsmittel extrahiert wird (vgl. z.B. Ullmanns Encyklopädie der technischen Chemie, 4. Auflage, Band 2, S. 722-729). Die Extraktion kann sowohl in der Kälte als auch in der Wärme erfolgen. Vorteilhaft ist die Extraktion in der Wärme, wobei die Temperatur jedoch so zu wählen ist, daß ein Zusammenbacken der vorzugsweise pulverförmigen Rohsalze vermieden wird. Besonders bevorzugt ist der Temperaturbereich von 40 bis 75 °C.

Die so gereinigten Salze haben einen höheren Metallgehalt (höhere Reinheit), wodurch im Vergleich zu den technischen Salzen eine geringere Menge benötigt wird, um die gleiche Wirkung als Kunststoffadditiv zu erzeugen. Der besondere Vorteil der nach der Extraktion gewonnenen Salze liegt jedoch in ihrem gegenüber den Rohsalzen deutlich verbesserten Farbverhalten bei der Anwendung als Additiv in Kunststoffen, was insbesondere durch die Anwendungsbeispiele 1 und 2 belegt wird.

Die Verwendung der nach dem erfindungsgemäßen Verfahren gereinigten Salze als Kunststoffadditive ist ebenfalls Gegenstand der vorliegenden Erfindung.

Hierzu müssen die beschriebenen Metallsalze lediglich homogen in dem gewünschten Polymer verteilt werden. Es kommt daher jede Mischmethode in Betracht, welche zu der angestrebten homogenen Verteilung der Komponenten führt. Nach einer Methode werden das feste, pulverförmige Polymer und das pulverförmige Metallsalz sowie gegebenenfalls noch weitere Additive und Zuschlagstoffe, wie Lichtschutzmittel, Verarbeitungsstabilisatoren, Gleit- und Trennmittel, Antistatika, Flammschutzmittel, Farbstoffe oder Pigmente, Treibmittel, Füll- und Verstärkungsstoffe oder Antioxidantien, durch Rühren in einem Banbury- oder Henschel-Mischer vermischt. Nach einer anderen Methode werden das Polymer und das Metallsalz sowie gegebenenfalls weitere Additive in einem Extruder unter Erwärmen geknetet und dann geeignet konfektioniert. Ebenfalls möglich ist die Herstellung von Konzentraten aus einem geeigneten Trägermaterial, d.h. in der Regel dem Polymer und den Metallsalzen, die später bei der Verarbeitung dem Kunststoff in einer Menge zugesetzt werden, welche zu der gewünschten Endkonzentration führt (Masterbatch-Technologie).

Die folgenden Einsatzmöglichkeiten sind von besonderer Bedeutung:

a) Verwendung als Gleit- und Trennmittel, insbesondere bei der Herstellung und Verarbeitung thermoplastischer oder duroplastischer Formmassen, wobei diese Formmassen 0,01 - 5 Gew.-%, vorzugsweise 0,1 - 1 Gew.-%, der Salze enthalten.

b) Verwendung als Säureakzeptor, insbesondere der Einsatz eines Calciummontanats als Säureakzeptor in Polyolefinen, wobei die Polyolefine 0,1 - 5 Gew.-% Calciummontanat enthalten.

c) Verwendung als Stabilisator, insbesondere die Verwendung eines Calcium- und/oder Zinkmontanats als Stabilisator für chlorhaltige Harze, bevorzugt für solche Harze, die durch Polymerisation von Vinylchlorid oder von Mischungen aus Vinylchlorid und Vinylacetat hergestellt werden.

Als speziellere Anwendungsgebiete sind zu nennen:

d) die Verwendung eines Calciummontanats als Gleitmittel und Säureakzeptor in Polyoxymethylen (POM),

e) die Verwendung eines Lithium- und/oder Calciummontanats als Gleit- und Nukleierungsmittel in Polyamiden und schließlich

f) die Verwendung eines Natriummontanats als Slip- und Nukleierungsmittel in thermoplastischen Polyestern (vgl. Ullmanns Encyklopädie der technischen Chemie, 4. Auflage, Band 15, S.253-273 (1978) und US-PS 3,619,267).

Die nachfolgenden Beispiele dienen zur Erläuterung der Erfindung.

Beispiel 1

155 g gemahlenes, rohes Natriummontanat (SZ 3,5 mg KOH/g; Na-Gehalt 5,0 Gew.-%) wurden in einem Kolben mit 620 g Methylethylketon bei 40 °C 30 min gerührt. Der ungelöste Anteil wurde abfiltriert und getrocknet. Man erhielt 132 g extrahiertes Natriummontanat (SZ 3,1 mg KOH/g, Na-Gehalt 5,8 Gew.-%). Die bei der Filtration erhaltene klare Lösung wurde eingeengt. Es verblieben 23,7 g eines wachsartigen Rückstands (SZ 16 mg KOH/g, VZ 38 mg KOH/g); SZ = Säurezahl, VZ = Verseifungszahl.

Beispiel 2

173,5 g gemahlenes, rohes Calciummontanat wurden mit 692 g Methylethylketon für 30 Minuten bei 40 °C gerührt. Der unlösliche Anteil wurde wie oben abfiltriert und getrocknet, das Filtrat wurde destillativ eingeengt.

Rückstand (extrahiertes Calciummontanat) 166,9 g (SZ 12 mg KOH/g, VZ 42 mg KOH/g) Extrakt 6,5 g (SZ 68 mg KOH/g, VZ 104 mg KOH/g)

Beispiel 3

173,5 g gemahlenes, rohes Calciummontanat wurden in einer Extraktionskolonne (Soxhlet) 3 Stunden lang mit Methylenchlorid unter Rückfluß extrahiert. Der unlösliche Rückstand wurde getrocknet, das Eluat wurde eingeengt.

Rückstand 162,5 g (SZ 15 mg KOH/g, VZ 52 mg KOH/g) Extrakt 11,3 g (SZ 33 mg KOH/g, VZ 81 mg KOH/g)

Anwendungsbeispiel 1

Eine Mischung aus 100 Teilen HDPE (MFI 190/21,6 ca 2 g/10 min), 0,8 Teilen Distearyl-thio-dipropionat und 8,0 Teilen Calciummontanat (Prüfprodukt) wurde auf einem Labor-Extruder bei einer Temperatureinstellung von 230 / 250 / 275 °C (Einzug-Düse) granuliert. Das Granulat wurde mit einem Farbmessgerät (Fa. Hunterlab) auf den Yellownessindex geprüft.

Um sicherzustellen, daß die extrahierten Bestandteile für die Gelbfärbung verantwortlich sind, wurde in einem Kontrollversuch das Calciummontanat durch die extrahierten Bestandteile ersetzt.

| Versuch | Prüfprodukt | Yellownessindex |
|---|---|---|
| A | Calciummontanat entspr. Beispiel 2 | + 30,7 |
| B | nicht extrahiertes Calciummontanat (Rohprodukt aus Beispiel 2) | + 36,2 |
| C | Extrakt aus Beispiel 2 | + 52,5 |

Anwendungsbeispiel 2

Polyvinylchlorid der Zusammensetzung

| | | |
|---|---|---|
| S-PVC, K-Wert ca. 58 | 100 | Teile |
| flüssiger Zinkstabilisator | 0,1 | " |
| Calciumstearat | 0,3 | " |
| Co-Stabilisator (ß-Diketon) | 0,3 | " |
| epox. Sojabohnenöl (ESO) | 3,0 | " |
| MBS-Schlagzähmacher | 8,0 | " |
| acrylathaltiges Verarbeitungshilfsmittel | 1,0 | " |
| Glycerinmonooleat | 1,0 | " |
| Schönungsmittel (Blaupigment) | 2,0 | " |
| Prüfprodukt (Calciummontanat) | 1,2 | " |

4

wurde auf einem Walzwerk bei 190°C 5 Minuten lang gewalzt. Danach wurden Proben des Fells entnommen und mit einem Farbmeßgerät (Fa. Hunterlab) auf ihren Yellownessindex geprüft.

| Versuch | Prüfprodukt | Yellownessindex |
|---------|-------------|-----------------|
| D | extrahiertes Calciummontanat entspr. Beispiel 2 | - 2,5 |
| E | nicht extrahiertes Calciummontanat (Rohprodukt aus Beispiel 2) | + 0,4 |

**Patentansprüche**

1. Verfahren zur Reinigung von Salzen langkettiger aliphatischer Carbonsäuren, dadurch gekennzeichnet, daß man die rohen Salze mit einem organischen Lösungsmittel extrahiert.

2. Verfahren gemäß Anspruch 1, dadurch gekennzeichnet, daß der Siedepunkt des als Extraktionsmittel dienenden organischen Lösungsmittels zwischen 30°C und 100°C liegt.

3. Verfahren gemäß Anspruch 1, dadurch gekennzeichnet, daß als Extraktionsmittel Methylethylketon verwendet wird.

4. Verfahren gemäß Anspruch 1, dadurch gekennzeichnet, daß die Salze langkettiger aliphatischer Carbonsäuren Salze der Elemente der I., II. oder III. Hauptgruppe oder der II., VII. oder VIII. Nebengruppe des Periodensystems der Elemente mit technischer Montansäure sind.

5. Verfahren gemäß Anspruch 1, dadurch gekennzeichnet, daß die Salze Lithium-, Natrium-, Calcium- oder Zinkmontanat oder deren Gemische sind.

6. Verfahren gemäß Anspruch 1, dadurch gekennzeichnet, daß das Salz Calciummontanat ist.

7. Verwendung der gemäß Anspruch 1 gereinigten Salze als Kunststoffadditive.

# Europäisches Patentamt

# EUROPÄISCHER RECHERCHENBERICHT

Nummer der Anmeldung

**EP 91 11 2597**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | GB-A-754 432   (HUGH HIGHGATE & CO.)<br>* Seite 5, Zeile 104 - Spalte 5, Zeile 119 *<br>– – – | 1,2 | C 07 C 53/126<br>C 07 C 51/41<br>C 08 K 3/24<br>C 11 D 13/02 |
| X | US-A-4 427 572   (J.B. AKERS ET AL.)<br>* Spalte 3, Zeile 59 - Spalte 3, Zeile 61 *<br>– – – | 1,2 | |
| X | GB-A-2 043 674   (UNILEVER)<br>* Seite 3, Zeile 57 - Seite 3, Zeile 58 *<br>– – – | 1,2 | |
| A | US-A-3 965 085   (B. HOLMBOM)<br>* Ansprüche 1, 6 *<br>– – – | 1 | |
| D,A | US-A-3 619 267   (K. WEISSERMEL ET AL.)<br>* Ansprüche 6-8 *<br>– – – – – | 7 | |

**RECHERCHIERTE SACHGEBIETE (Int. Cl.5)**

C 07 C
C 08 K
C 11 D

Der vorliegende Recherchenbericht wurde für alle Patentansprüche erstellt

| Recherchenort | Abschlußdatum der Recherche | Prüfer |
|---|---|---|
| Berlin | 13 November 91 | PROBERT C.L. |